(19)

Europäisches
Patentamt
European
Patent Office
Office européen
des brevets

(11) **EP 4 342 442 A1**

(12) **EUROPEAN PATENT APPLICATION**

(43) Date of publication:
**27.03.2024 Bulletin 2024/13**

(21) Application number: **23192545.4**

(22) Date of filing: **22.08.2023**

(51) International Patent Classification (IPC):
**A61K 8/00** *(2006.01)*    **A61K 36/07** *(2006.01)*
**A61K 36/65** *(2006.01)*    **A61K 36/8984** *(2006.01)*
**A61P 17/00** *(2006.01)*    **A61P 17/02** *(2006.01)*
**A61P 17/06** *(2006.01)*

(52) Cooperative Patent Classification (CPC):
(C-Sets available)
**A61K 36/07; A61K 8/9728; A61K 8/9789;**
**A61K 8/9794; A61K 36/65; A61K 36/8984;**
**A61P 17/00; A61P 17/02; A61P 17/06;**
**A61Q 19/00; A61Q 19/02;** A61K 2236/333;
A61K 2236/51; A61K 2800/782; A61K 2800/84;

(Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **25.08.2022 CN 202211026791**

(71) Applicant: **Infinitus (China) Company Ltd.**
**Jiangmen, Guangdong 529156 (CN)**

(72) Inventors:
 • **LIU, Xiaoying**
 **Jiangmen, 529156 (CN)**

 • **TAI, Meiling**
 **Jiangmen, 529156 (CN)**
 • **CHE, Biao**
 **Jiangmen, 529156 (CN)**
 • **DU, Zhiyun**
 **Jiangmen, 529156 (CN)**
 • **LIN, Li**
 **Jiangmen, 529156 (CN)**
 • **HAN, Ping**
 **Jiangmen, 529156 (CN)**

(74) Representative: **Kehl, Ascherl, Liebhoff & Ettmayr**
**Patentanwälte Partnerschaft mbB**
**Emil-Riedel-Straße 18**
**80538 München (DE)**

(54) **COMPOSITION WITH ENDOTHELIN INHIBITING EFFECT, PREPARATION METHOD AND USE THEREOF**

(57) The present disclosure discloses a composition with endothelin inhibiting effect, a preparation method and the use thereof. The composition comprises an extract of *Paeonia latiflora* Pall., *Dendrobium, and Tricholoma matsutake* (S. Ito & S. Imai) Singer. A composition obtained in the present disclosure by compounding the extract of *Paeonia latiflora* Pall., *Dendrobium,* and *Tricholoma matsutake* (S. Ito & S. Imai) Singer, has a good inhibitory effect on endothelin, an inhibition rate of up to 19.08-47.51% against endothelin in skin; in addition, the composition is made up of all natural extracts, has no obvious toxic and side effects, is relatively inexpensive, and has greater application prospects.

FIG. 1

(52) Cooperative Patent Classification (CPC): (Cont.)
Y02A 50/30

C-Sets
**A61K 36/07, A61K 2300/00;**
**A61K 36/65, A61K 2300/00;**
**A61K 36/8984, A61K 2300/00**

**Description**

**TECHNICAL FIELD**

The present disclosure belongs to the field of pharmaceutical compositions, and in particular relates to a composition with endothelin inhibiting effect, a preparation method therefor and the use thereof.

**BACKGROUND**

Endothelin (ET), a 21-amino-acid polypeptide with vasoconstrictive function in the human body, is widely expressed in most tissues of the human body. Endothelin is also highly expressed in skin keratinocytes and fibroblasts, which is also an endogenous injury factor. ET-1 (endothelin-1) regulates local vascular tone by paracrine, and it can directly promote the proliferation of vascular endothelial cells and can also interact with vascular endothelial growth factor (VEGF). ET-1 has the characteristics of promoting cell growth and mitosis and is an inflammatory factor that causes various diseases. It has been found from relevant studies that endothelin is closely related to cardiovascular and cerebrovascular diseases, pulmonary arterial hypertension, retinopathy and tumors. Endothelin affects skin wound healing and scar formation and is also involved in the regulation of skin diseases such as lupus erythematosus, melanoma and pigmentation. Therefore, effective regulation of endothelin production is of great significance to the human health.
At present, it has been found that peptide ET antagonists with strong effects include BQ123, BQ160, FR2139317, etc. However, due to the lower oral bioavailability of peptide ET antagonists, the application thereof in related fields is greatly limited. Among the discovered non-peptide antagonists, bosentan, tezosentan, ambrisentan, etc. have been marketed. These non-peptide ET antagonists have achieved good effects in the treatment of hypertension, pulmonary arterial hypertension, myocardial infarction, cerebral vasospasm, etc. However, these non-peptide ET antagonists all have certain hypersensitivity or toxic and side effects on the human body, e.g., heart failure and hepatic dysfunction. However, endothelin antagonists containing purely natural plant extracts are still less, and in particular, active substances for topical endothelin antagonists against skin problems are rarely found.
Skin keratinocytes can release the signal factor endothelin ET-1. When the skin is irradiated by ultraviolet radiation, keratinocytes will release more endothelin ET-1. After receiving this signal factor, melanocytes will differentiate and proliferate, accelerate the synthesis of melanin, and thus make melanin increase sharply. Endothelin antagonist refers to substances that can antagonize endothelin ET-1, which can inhibit the production of endothelin ET-1 in keratinocytes and fundamentally cut off the transmission of melanin granules from melanocytes to skin epidermis, thereby restoring the speed of melanin synthesis in each melanocyte to a normal level, avoiding uneven or excessive accumulation of melanin locally due to the rapid production of melanin.
Therefore, it is of great significance to develop more purely natural plant extract compositions having endothelin inhibiting effect with stable effects, less toxic and side effects and relatively low price, which can be used for preventing and treating skin diseases in which endothelin is taken as a target, e.g., lupus erythematosus, melanoma and pigmentation.

**SUMMARY**

The present disclosure discloses a composition with endothelin ET-1 inhibiting effect, a preparation method therefor and the use thereof. The composition of the present disclosure comprises an extract of *Paeonia latiflora* Pall. or *Paeonia veitchii* Lynch, an extract of *Dendrobium,* and an extract of *Tricholoma matsutake* (S. Ito & S. Imai) Singer at a specific proportioning ratio. The composition obtained in the present disclosure by combining the extract of *Paeonia latiflora* Pall., the extract of *Dendrobium,* and the extract of *Tricholoma matsutake* (S. Ito & S. Imai) Singer at a specific ratio has a good inhibitory effect on ET-1 and a potency obviously superior to each component alone or a combination of simply added two components. It can generate significant synergistic effects by adjusting the proportion. The three raw materials in the present disclosure are all purely natural Chinese medicines, with high safety and relatively low costs. They can be used as either active ingredients to effectively prevent and treat skin endothelin regulation-associated problems such as speckle, scleroderma, psoriasis, and wound healing or as active ingredients to prepare medicaments for effectively relieving or treating diseases such as cardiovascular and cerebrovascular diseases, tumors, and pulmonary arterial hypertension.
In first aspect of the present disclosure, it provids a composition with endothelin antagonistic effect, which comprises an extract of *Paeonia latiflora* Pall. or *Paeonia veitchii* Lynch, an extract of *Dendrobium,* and an extract of *Tricholoma matsutake* (S. Ito & S. Imai) Singer.
In some embodiments of the present disclosure, a mass ratio of the extract of *Paeonia latiflora* Pall., the extract of *Dendrobium* and the extract of *Tricholoma matsutake* (S. Ito & S. Imai) Singer is (1-5) : (1-5) : (1-3).
In some embodiments of the present disclosure, the mass ratio of the extract of *Paeonia latiflora* Pall., the extract of *Dendrobium* and the extract of *Tricholoma matsutake* (S. Ito & S. Imai) Singer is (1.5-3) : (1.5-3) : (1-2).

In the present disclosure, the inventors have found that the above-mentioned composition can exert a better inhibitory effect on ET-1 only in the case of a specific ratio, indicating that there is a complementary enhancement effect between the components in the above-mentioned composition at a specific ratio.

In some embodiments of the present disclosure, the mass ratio of the extract of *Paeonia latiflora* Pall., the extract of *Dendrobium* and the extract of *Tricholoma matsutake* (S. Ito & S. Imai) Singer is 2 : 2 : 1.

In specific embodiments of the present disclosure, the inventors have found that based on the mass ratio of the extract of *Paeonia latiflora* Pall., the extract of *Dendrobium* and the extract of *Tricholoma matsutake* (S. Ito & S. Imai) Singer of 2 : 2 : 1, the above-mentioned composition can exert the best inhibitory effect on ET-1, which is significantly higher than that of any other combination with any ratio. It was indicated that the components in the above-mentioned composition have the best complementary enhancement effect at a ratio of 2 : 2 : 1. The main active ingredients in the composition include paeoniflorin, matsutakeol, matsutake polysaccharides, dendrobium polysaccharides, etc.

In some embodiments of the present disclosure, the *Dendrobium* comprises *Dendrobium nobile* Lindl. and *Dendrobium officinale..*

In some embodiments of the present disclosure, a preparation method for the extract of *Paeonia latiflora* Pall. includes soaking raw material of *Paeonia latiflora* Pall. in 70-95% ethanol aqueous solution, and performing reflux extraction at 60-85°C to obtain the extract of *Paeonia latiflora* Pall.,

wherein a mass of a raw material of *Paeonia latiflora* Pall. to a volume of the 70-95% ethanol aqueous solution (i.e., a material-to-liquid ratio) is 1 mg : 10-20 mL.

In some embodiments of the present disclosure, the raw material of *Paeonia latiflora* Pall. includes *Paeonia latiflora* Pall. roots.

Of course, those skilled in the art can rationally select and use other raw materials of *Paeonia latiflora* Pall. according to actual use requirements, including but not limited to the above-mentioned *Paeonia latiflora* Pall. roots.

In some embodiments of the present disclosure, the preparation method for the extract of *Paeonia latiflora* Pall. involves soaking the raw material of *Paeonia latiflora* Pall. in 70-95% ethanol aqueous solution for 0.5-1 h, and performing reflux extraction at 60-85°C for 1.5-3 h, followed by filtration, concentration and drying to obtain the extract of *Paeonia latiflora* Pall..

In some embodiments of the present disclosure, the raw material of *Paeonia latiflora* Pall. is subjected to a crushing treatment. It is crushed and passed through a 40-80-mesh sieve.

In some embodiments of the present disclosure, the *Paeonia latiflora* Pall. roots are crushed and passed through a 60-mesh sieve, an 85% ethanol aqueous solution is added for soaking for 1 h, and the mixture is subjected to reflux extraction at 80°C for 3 h, followed by filtration for a particle size of 0.5-5 μm, concentration by rotary evaporation, and drying to obtain the extract of *Paeonia latiflora* Pall..

In some embodiments of the present disclosure, the material-to-liquid ratio of the *Paeonia latiflora* Pall. roots to the 85% ethanol aqueous solution is 1 (mg) : 15 (mL).

In some embodiments of the present disclosure, a preparation method for the extract of *Dendrobium* includes..adding water to soak a raw material of *Dendrobium nobile* Lindl., performing reflux extraction at 95-100°C, filtering the extraction product, and freeze-drying the filtrate to obtain the extract of *Dendrobium nobile* Lindl.

In the method, the material-to-liquid ratio of the raw material of *Dendrobium nobile* Lindl. to water is 1 mg : 10-20 mL.

In some embodiments of the present disclosure, the drying is freeze drying.

In some embodiments of the present disclosure, the preparation method for the extract of *Dendrobium* includes soaking raw material of *Dendrobium nobile* Lindl. in water for 0.5-2 h, heating the mixture to boiling for extraction for 1.5-3 h, filtering the extraction product, then taking the filtrate, and concentrating and freeze-drying the filtrate for 36-72 h to obtain the extract of *Dendrobium.*

In some embodiments of the present disclosure, the raw material of *Dendrobium nobile* Lindl. is subjected to a crushing treatment. It is crushed and passed through a 50-100-mesh sieve.

In some embodiments of the present disclosure, the filtration is filtration through a 90-110-mesh filter cloth.

In some embodiments of the present disclosure, the filtration is filtration through a 100-mesh filter cloth.

In some embodiments of the present disclosure, the material-to-liquid ratio of the raw material of *Dendrobium nobile* Lindl. to water is 1 mg : 15 mL.

In some embodiments of the present disclosure, the preparation method for the extract of *Dendrobium* involves crushing raw material of *Dendrobium nobile* Lindl., passing the crushed raw material through an 80-mesh sieve, mixing the *Dendrobium nobile* Lindl. with water at a material-to-liquid ratio of 1 (mg) : 15 (mL), soaking the mixture for 1 h, heating the mixture to boiling, then extracting the mixture for 2 h, filtering the extraction product with a 100-mesh filter cloth, collecting the filtrate, subjecting the filtrate to concentration by rotary evaporation under reduced pressure, and freeze-drying the concentration product for 48 h to obtain the extract of *Dendrobium.*

In some embodiments of the present disclosure, a preparation method for the extract of *Dendrobium* involves mixing a raw material of *Dendrobium nobile* Lindl. with *Lactobacillus sp.* for fermentation to obtain the extract of *Dendrobium.*

In some embodiments of the present disclosure, the *Lactobacillus sp.* includes *Lactobacillus reuteri.*

In some embodiments of the present disclosure, the *Lactobacillus reuteri* is *Lactobacillus reuteri* CCFM8631, which can be purchased from China General Microbiological Culture Collection Center (CGMCC).

In some embodiments of the present disclosure, before mixing and fermentation, a fermentation auxiliary material is further added during the preparation method.

In some embodiments of the present disclosure, the fermentation auxiliary material includes a *Lactobacillus* proliferation factor, a trace element, and a cosolvent.

In some embodiments of the present disclosure, the *Lactobacillus* proliferation factor comprises at least one of yeast extract powder, yeast extract, yeast peptone, tryptone and soybean peptone.

In some embodiments of the present disclosure, the trace element is at least one of magnesium sulfate and manganese sulfate.

In some embodiments of the present disclosure, the trace element is the combination of magnesium sulfate and manganese sulfate.

In one embodiment of the present disclosure, the cosolvent includes Tween-80.

In some embodiments of the present disclosure, a mixing mass ratio of the raw material of *Dendrobium nobile* Lindl., the *Lactobacillus* proliferation factor and the trace element is 40-80 : 10-40 : 0.1-1.0.

In some embodiments of the present disclosure, the mixing mass ratio of the raw material of *Dendrobium nobile* Lindl., the *Lactobacillus* proliferation factor and the trace element is 40-60 : 10-25 : 0.25-0.83.

In some embodiments of the present disclosure, the preparation method for the extract of *Dendrobium* specifically involves mixing raw material of *Dendrobium nobile* Lindl., yeast extract powder, magnesium sulfate heptahydrate, manganese sulfate, Tween-80 and water, then adding *Lactobacillus reuteri,* and performing fermentation to obtain the extract of *Dendrobium.*

In one embodiment of the present disclosure, a pH of the fermentation system is 5-7.

In one embodiment of the present disclosure, the pH of the fermentation system is 6-7.

In one embodiment of the present disclosure, the effective viable bacteria count of *Lactobacillus reuteri* is $1.0 \times 10^5$ to $5.0 \times 10^8$ cfu/mL.

In one embodiment of the present disclosure, the fermentation time is 16-24 h.

In one embodiment of the present disclosure, the fermentation temperature is 35-37°C.

In one embodiment of the present disclosure, before the addition of *Lactobacillus reuteri* for fermentation, the final concentration of *Dendrobium* in the system is 40-80 g/L, the final concentration of the yeast extract powder is 10-40 g/L, the final concentration of magnesium sulfate heptahydrate is 0.48-0.58 g/L, the final concentration of manganese sulfate is 0.15-0.25 g/L, and the final concentration of Tween-80 is 0.08-0.1% (v/v).

In one embodiment of the present disclosure, before the addition of *Lactobacillus reuteri* for fermentation, the final concentration of *Dendrobium* in the system is 40-80 g/L, the final concentration of the yeast extract powder is 10-40 g/L, the final concentration of magnesium sulfate heptahydrate is 0.58 g/L, the final concentration of manganese sulfate is 0.25 g/L, and the final concentration of Tween-80 is 0.1% (v/v).

In the present disclosure, the inventors have found that the extract of *Dendrobium* obtained after fermentation with the *Lactobacillus* proliferation factor can produce similar effects to the extract of *Dendrobium* obtained by extraction with water.

In some embodiments of the present disclosure, a preparation method for the extract of *Tricholoma matsutake* (S. Ito & S. Imai) Singer involves reflux extraction of raw material of *Tricholoma matsutake* (S. Ito & S. Imai) Singer in 40-70% ethanol aqueous solution at 40-80°C to obtain the extract of *Tricholoma matsutake* (S. Ito & S. Imai) Singer, wherein a mass of a raw material of *Tricholoma matsutake* (S. Ito & S. Imai) Singer to a volume of the 40-70% ethanol aqueous solution (i.e., a material-to-liquid ratio) is 1 mg : 10-30 mL.

In some embodiments of the present disclosure, the preparation method for the extract of *Tricholoma matsutake* (S. Ito & S. Imai) Singer involves subjecting a raw material of *Tricholoma matsutake* (S. Ito & S. Imai) Singer to reflux extraction in a 40-70% ethanol aqueous solution at 40-80°C for 1-4 h, wherein the material-to-liquid ratio of the *Tricholoma matsutake* (S. Ito & S. Imai) Singer to the ethanol aqueous solution is 1 (mg) : 10-30 (mL); and after filtration, taking the filtrate, and subjecting the filtrate to concentration and drying to obtain the extract of *Tricholoma matsutake* (S. Ito & S. Imai) Singer.

In some embodiments of the present disclosure, the raw material of *Tricholoma matsutake* (S. Ito & S. Imai) Singer is subjected to a crushing treatment.

In some embodiments of the present disclosure, the filtration is filtration through a 80-120-mesh filter cloth.

In some embodiments of the present disclosure, the filtration is filtration through a 100-mesh filter cloth.

In some embodiments of the present disclosure, the preparation method for the extract of *Tricholoma matsutake* (S. Ito & S. Imai) Singer involves crushing the raw material of *Tricholoma matsutake* (S. Ito & S. Imai) Singer, then subjecting the crushed raw material to reflux extraction in 50% ethanol aqueous solution at 50°C for 3 h, wherein the material-to-liquid ratio of the raw material of *Tricholoma matsutake* (S. Ito & S. Imai) Singer to the 50% ethanol aqueous solution is 1 (mg) : 20 (mL); and after filtration through a 100-mesh filter cloth, collecting the filtrate, and subjecting the filtrate to concentration by rotary evaporation and then drying to obtain the extract of *Tricholoma matsutake* (S. Ito & S. Imai) Singer.

In the present disclosure, the inventors have found that the extracts of *Paeonia latiflora* Pall., *Tricholoma matsutake* (S. Ito & S. Imai) Singer and *Dendrobium* obtained by the above-mentioned preparation methods can play a better complementary enhancement effect and have a better effect as compared with a combination of mixed extracts of *Paeonia latiflora* Pall., *Tricholoma matsutake* (S. Ito & S. Imai) Singer and *Dendrobium* obtained in the comparative examples in the present disclosure or by a general existing extraction method.

In second aspect of the present disclosure, it provides the use of the composition described in the first aspect of the present disclosure in inhibiting endothelin.

In some embodiments of the present disclosure, the endothelin inhibitor is used for inhibiting the expression of endothelin and endothelin-related genes.

In some embodiments of the present disclosure, the dosage forms of the endothelin inhibitor include, but are not limited to, solutions, powders, lyophilizers, capsules, tablets, pills, granules, decoctions, and syrups. Those skilled in the art would rationally select an appropriate dosage form according to actual use requirements.

In third aspect of the present disclosure, it provides the use of the composition described in the first aspect of the present disclosure for use in brightening and whitening or for reducing melanin or tyrosinase.

In the present disclosure, through animal experiments, the inventors have found that the composition described in the first aspect of the present disclosure can significantly ameliorate melanin synthesis and accumulation caused by UVB (ultraviolet radiation b), and the use effect is better than that of the extract of *Paeonia latiflora* Pall., *Dendrobium* or *Tricholoma matsutake* (S. Ito & S. Imai) Singer alone.

In fourth aspect of the present disclosure, it provides a food, medicament or cosmetic product, which includes the composition described in the first aspect of the present disclosure.

In the present disclosure, the extracts of *Paeonia latiflora* Pall., *Dendrobium, Tricholoma matsutake* (S. Ito & S. Imai) Singer, etc. are all derived from natural plants, and no organic solvent is used during extraction. The composition composed of these extracts is non-toxic and harmless and relatively safe for the human body and can meet the use requirements and regulatory standards of general products such as food, medicament or cosmetic products.

In some embodiments of the present disclosure, the food, medicament or cosmetic product further includes an auxiliary material.

In some embodiments of the present disclosure, the auxiliary material includes food additives, food raw materials, pharmaceutically acceptable carriers, pharmaceutically acceptable excipients, conventional cosmetic additives, or base preparations.

In some embodiments of the present disclosure, the auxiliary material is a cosmetic base emulsion.

In the present disclosure, the inventors used a cosmetic base emulsion as a carrier to test the actual use efficacy of the composition in the present disclosure and have found that the composition described in the first aspect of the present disclosure can significantly ameliorate melanin synthesis and accumulation caused by UVB, and the use effect is better than that of the extract of *Paeonia latiflora* Pall., *Dendrobium* or *Tricholoma matsutake* (S. Ito & S. Imai) Singer alone.

In some embodiments of the present disclosure, the formula of the cosmetic base emulsion is as shown in Table 2 in the description.

In fifth aspect of the present disclosure, it provides the use of the composition described in the first aspect of the present disclosure for use as a medicament.

In some embodiments of the present disclosure, the medicament can prevent and/or ameliorate related diseases and conditions in which endothelin is taken as a target.

In some embodiments of the present disclosure, the related diseases and conditions in endothelin is taken as a target include, but are not limited to, skin endothelin regulation-associated problems such as speckle, scleroderma, psoriasis, and wound healing, and diseases such as cardiovascular and cerebrovascular diseases, tumors, and pulmonary arterial hypertension.

In some embodiments of the present disclosure, the composition is used as the main active substance in the medicament.

In some embodiments of the present disclosure, the composition is used as the only active substance in the medicament.

In some embodiments of the present disclosure, the medicament further contains a pharmaceutically acceptable excipient.

In some embodiments of the present disclosure, the pharmaceutically acceptable excipient includes, but is not limited to, preservatives, flavoring agents, colorants, suspending agents, wetting agents, emulsifiers, pH regulators, antioxidants, bacteriostats, fillers, diluents, binders, disintegrants, lubricants, coating materials (such as slow-release coatings and enteric-coated coatings), thickeners and absorption enhancers.

In some embodiments of the present disclosure, the dosage forms of the medicament include but are not limited to injections, tablets, capsules, oral liquids, granules and aerosols.

In sixth aspect of the present disclosure, it provides the composition described in the first aspect of the present disclosure for use in reducing melanin or tyrosinase in skin.

In seventh aspect of the present disclosure, it provides the composition described in the first aspect of the present disclosure for use in preventing and/or ameliorating a disease in which endothelin is taken as a target.

In some embodiments of the present disclosure, the disease is skin endothelin regulation-associated problems comprising speckle, scleroderma, psoriasis, and wound healing, and diseases comprising cardiovascular and cerebrovascular diseases, tumors, and pulmonary arterial hypertension.

Beneficial Effects of the Disclosure:

1. The composition obtained in the present disclosure by compounding the extract of *Paeonia latiflora* Pall., the extract of *Dendrobium nobile* Lindl., and the extract of *Tricholoma matsutake* (S. Ito & S. Imai) Singer, which are obtained by specific methods, has a good inhibitory effect on endothelin. The potency obviously superior to each component alone or a combination of randomly compounded or simply added multiple components, the inhibition rate of up to 19.08-47.51% against endothelin in skin, which shows a significant synergistic effect.

2. The compositions in the present disclosure are all obtained by combining natural plant extracts, and the three extracts in the composition are all extracted from purely natural Chinese medicines, with lower toxic and side effects, less harm to the human body, relatively low price and greater application prospects.

## BRIEF DESCRIPTION OF DRAWINGS

Fig. 1 is a staining diagram (200×) of melanin in the skin of the back of mice in different groups after 4 weeks.

Fig. 2 is a comparison diagram of the integrated optical density (IOD) values of melanin in the skin of the back of mice in different groups after 4 weeks.

## DETAILED DESCRIPTION

In order to make the object, technical solution and technical effects of the present disclosure clearer, the present disclosure will be further illustrated below in detail in conjunction with specific embodiments. It should be understood that the specific embodiments described in the present description are only intended for explaining, rather than limiting, the present disclosure.

Unless otherwise specified, the experimental materials and reagents used are all commercially available conventional consumables and reagents.

In the following examples, the plant extracts used were all obtained based on the following preparation methods.

The preparation method for the extract of *Paeonia latiflora* Pall. involved:

taking and crushing dried *Paeonia latiflora* Pall. roots and passing the crushed product through a 60-mesh sieve; and then adding an 85% ethanol aqueous solution for soaking for 1 h and subjecting the mixture to reflux extraction at 80°C for 3 h, wherein the material-to-liquid ratio of the raw material (powder) of the *Paeonia latiflora* Pall. roots to the 85% ethanol aqueous solution was 1 (mg) : 15 (mL). After the reflux extraction was completed, the extraction liquid of the *Paeonia latiflora* Pall. roots was filtered by cardboard filtration (the filtration particle size was 0.5-5 μm) to remove impurities, and the filtrate was subjected to concentration by rotary evaporation and then drying to obtain the extract of *Paeonia latiflora* Pall. The mass ratio of the final extract to the raw material was 13.58%.

The preparation method for the extract of *Tricholoma matsutake* (S. Ito & S. Imai) Singer involved:

taking and crushing *Tricholoma matsutake* (S. Ito & S. Imai) Singer, then subjecting the crushed material to reflux extraction in a 50% ethanol aqueous solution at 50°C for 3 h, wherein the material-to-liquid ratio of the *Tricholoma matsutake* (S. Ito & S. Imai) Singer (powder) to the 50% ethanol aqueous solution was 1 (mg) : 20 (mL); and after the reflux extraction was completed, passing the extraction product through a 100-mesh filter cloth, collecting the filtrate, and subjecting the filtrate to concentration by rotary evaporation and then drying to obtain the extract of *Tricholoma matsutake* (S. Ito & S. Imai) Singer. The mass ratio of the final extract to the raw material was 12.43%.

The preparation method for the extract of *Dendrobium* involved:

taking and crushing dried *Dendrobium nobile* Lindl., passing the crushed material through an 80-mesh sieve, mixing the *Dendrobium nobile* Lindl. (powder) with water at a material-to-liquid ratio of 1 (mg) : 15 (mL), soaking the mixture for 1 h, heating the mixture to boiling, then extracting the mixture for 2 h, filtering the extraction product with a 100-mesh filter cloth, and collecting the filtrate; and subjecting the filtrate to concentration by rotary evaporation under reduced pressure, and freeze-drying the concentration product for 48 h to finally obtain the extract of *Dendrobium*. The mass ratio of the final extract to the raw material was 19.12%.

**EXAMPLE 1**

The composition in this example was obtained by mixing the extract of *Paeonia latiflora* Pall., the extract of *Dendrobium nobile* Lindl., and the extract of *Tricholoma matsutake* (S. Ito & S. Imai) Singer at a mass ratio of 2 : 2 : 1. In this example, based on the total mass of the composition, water was added according to a material-to-liquid ratio of 1 mg : 1 mL to prepare a solution for later use.

**EXAMPLE 2**

The composition in this example was obtained by mixing the extract of *Paeonia latiflora* Pall., the extract of *Dendrobium nobile* Lindl., and the extract of *Tricholoma matsutake* (S. Ito & S. Imai) Singer at a mass ratio of 5 : 2 : 1. In this example, based on the total mass of the composition, water was added according to a material-to-liquid ratio of 1 mg : 1 mL to prepare a solution for later use.

**EXAMPLE 3**

The composition in this example was obtained by mixing the extract of *Paeonia latiflora* Pall., the extract of *Dendrobium nobile* Lindl., and the extract of *Tricholoma matsutake* (S. Ito & S. Imai) Singer at a mass ratio of 1 : 5 : 3. In this example, based on the total mass of the composition, water was added according to a material-to-liquid ratio of 1 mg : 1 mL to prepare a solution for later use.

**COMPARATIVE EXAMPLE 1**

This example was prepared according to the method in Example 1, wherein only the extract of *Paeonia latiflora* Pall., the mass of which was the same as the total mass of the composition in Example 1, was added to the composition.

**COMPARATIVE EXAMPLE 2**

This example was prepared according to the method in Example 1, wherein only the extract of *Dendrobium nobile* Lindl., the mass of which was the same as the total mass of the composition in Example 1, was added to the composition.

**COMPARATIVE EXAMPLE 3**

This example was prepared according to the method in Example 1, wherein only the extract of *Tricholoma matsutake* (S. Ito & S. Imai) Singer, the mass of which was the same as the total mass of the composition in Example 1, was added to the composition.

**COMPARATIVE EXAMPLE 4**

This example was prepared according to the method in Example 1, wherein only the extract of *Paeonia latiflora* Pall. and the extract of *Dendrobium nobile* Lindl. at a mass ratio of 1 : 1 were added to the composition.

**COMPARATIVE EXAMPLE 5**

This example was prepared according to the method in Example 1, wherein only the extract of *Paeonia latiflora* Pall. and the extract of *Tricholoma matsutake* (S. Ito & S. Imai) Singer at a mass ratio of 1 : 1 were added to the composition.

**COMPARATIVE EXAMPLE 6**

This example was prepared according to the method in Example 1, wherein only the extract of *Dendrobium nobile* Lindl. and the extract of *Tricholoma matsutake* (S. Ito & S. Imai) Singer at a mass ratio of 1 : 1 were added to the composition.

## COMPARATIVE EXAMPLE 7

This example was prepared according to the method in Example 1, wherein only the extract of *Paeonia latiflora* Pall., the extract of *Dendrobium nobile* Lindl. and the extract of *Tricholoma matsutake* (S. Ito & S. Imai) Singer at a mass ratio of 1 : 1 : 5 were added to the composition.

## COMPARATIVE EXAMPLE 8

In this comparative example, in the preparation method for the extract of *Paeonia latiflora* Pall., a 50% ethanol aqueous solution was used to replace the 85% ethanol aqueous solution in the above example; in the preparation method for the extract of *Tricholoma matsutake* (S. Ito & S. Imai) Singer, a 95% ethanol aqueous solution was used to replace the 50% ethanol aqueous solution; and in the preparation method for the extract of *Dendrobium nobile* Lindl., the material-to-liquid ratio of 1 (mg) :15 (mL) was adjusted to 1 (mg) : 8 (mL), and after concentration by rotary evaporation, drying in an oven at 65°C was used to replace freeze-drying.

This example was prepared according to the method in Example 1, wherein only the extract of *Paeonia latiflora* Pall., the extract of *Dendrobium nobile* Lindl. and the extract of *Tricholoma matsutake* (S. Ito & S. Imai) Singer at a mass ratio of 2 : 2 : 1 were added to the composition.

## COMPARATIVE EXAMPLE 9

The extraction method for each plant extract was the same as in Comparative Example 8. This example was prepared according to the method in Example 1, wherein only the extract of *Paeonia latiflora* Pall., the extract of *Dendrobium nobile* Lindl. and the extract of *Tricholoma matsutake* (S. Ito & S. Imai) Singer at a mass ratio of 5 : 2 : 1 were added to the composition.

**Test Example 1: Inhibitory effect of composition on expression of ET-1 in skin keratinocytes HaCaT**

(1) Construction of skin keratinocytes HaCaT:

Immortalized human epidermal cells (HaCaT cells) were inoculated into a DMEM medium containing 10% fetal bovine serum and 1% double antibiotic combination (antibiotics) and cultured at 37°C and 5% $CO_2$. The cultured HaCaT cells were digested and detached with 0.25% insulin-EDTA, and the cells were cultured in a 96-well plate at a concentration of $5 \times 10^3$ cells/well. Complete culture media containing the compositions of Examples 1-3 and Comparative Examples 1-9 were added separately, wherein the final concentration of the composition in the final system of each group was 200 $\mu$g/mL. Culturing was continued for 48 h. After the incubation was completed, cells were collected for ELISA to detect the expression of ET-1 (for ELISA detection, reference can be made to the use instructions of the ET-1 ELISA detection kit). The test was carried out in triplicate in parallel. A blank control group was set up, in which an equal amount of a complete medium without any treatment was added. Calculation of cell inhibition rate:

$$\text{Inhibition rate} = \frac{\text{Blank control group} - \text{Experimental group}}{\text{Blank control group}}$$

The experimental data were analyzed for significance.
The results were as shown in Table 1.

Table 1: Inhibitory effect of composition on expression of ET-1 in skin keratinocytes HaCaT

| Grouping | Composition (mass ratio) | ET-1 content (pg/mg) | Inhibition rate (%) |
|---|---|---|---|
| Control group | \ | 9.17±1.02 | \ |
| EXAMPLE 1 | *Paeonia latiflora* Pall. : *Dendrobium nobile* Lindl. : *Tricholoma matsutake* (S. Ito & S. Imai) Singer = 2 : 2 : 1 | 6.51±0.26****Δ Δ Δ Δ | 29.02 |
| EXAMPLE 2 | *Paeonia latiflora* Pall. : *Dendrobium nobile* Lindl. : *Tricholoma matsutake* (S. Ito & S. Imai) Singer = 5 : 2 : 1 | 6.61±0.25****Δ Δ Δ Δ | 27.88 |

(continued)

| Grouping | Composition (mass ratio) | ET-1 content (pg/mg) | Inhibition rate (%) |
|---|---|---|---|
| EXAMPLE 3 | *Paeonia latiflora* Pall. : *Dendrobium nobile* Lindl. : *Tricholoma matsutake* (S. Ito & S. Imai) Singer = 1 : 5 : 3 | $7.42\pm0.09$****$\Delta\Delta$ | 19.08 |
| COMPARATIVE EXAMPLE 1 | *Paeonia latiflora* Pall. | $8.36\pm0.19$** | 8.82 |
| COMPARATIVE EXAMPLE 2 | *Dendrobium nobile* Lindl. | $8.34\pm0.11$** | 9.08 |
| COMPARATIVE EXAMPLE 3 | *Tricholoma matsutake* (S. Ito & S. Imai) Singer | $8.23\pm0.10$*** | 10.26 |
| COMPARATIVE EXAMPLE 4 | *Paeonia latiflora* Pall. : *Dendrobium nobile* Lindl. = 1 : 1 | $8.25\pm0.07$*** | 10.06 |
| COMPARATIVE EXAMPLE 5 | *Paeonia latiflora* Pall. : *Tricholoma matsutake* (S. Ito & S. Imai) Singer = 1 : 1 | $8.42\pm0.26$** | 8.18 |
| COMPARATIVE EXAMPLE 6 | *Dendrobium nobile* Lindl. : *Tricholoma matsutake* (S. Ito & S. Imai) Singer = 1 : 1 | $8.20\pm0.17$*** | 10.61 |
| COMPARATIVE EXAMPLE 7 | *Paeonia latiflora* Pall. : *Dendrobium nobile* Lindl. : *Tricholoma matsutake* (S. Ito & S. Imai) Singer = 1 : 1 : 5 | $8.51\pm0.23$* | 7.2 |
| COMPARATIVE EXAMPLE 8 | *Paeonia latiflora* Pall. : *Dendrobium nobile* Lindl. : *Tricholoma matsutake* (S. Ito & S. Imai) Singer = 2 : 2 : 1 (different process) | $8.43\pm0.24$** | 8.11 |
| COMPARATIVE EXAMPLE 9 | *Paeonia latiflora* Pall. : *Dendrobium nobile* Lindl. : *Tricholoma matsutake* (S. Ito & S. Imai) Singer = 5 : 2 : 1 (different process) | $8.41\pm0.19$** | 8.32 |
| * represented a significant difference between each group and the blank control group, wherein *$P < 0.05$, **$P < 0.01$, ***$< 0.001$, and ****$< 0.0001$. $\Delta$ represented a significant difference between each example and Comparative Example 6 (Comparative Example 6 had the best effect among the comparative examples), wherein $\Delta P < 0.05$, $\Delta\Delta P < 0.01$, $\Delta\Delta\Delta P < 0.001$, and $\Delta\Delta\Delta\Delta P < 0.0001$. | | | |

From the content of ET-1 in different experimental groups shown in Table 1, it could be found that the expression of ET-1 in all the groups in which the compositions of the examples were used was significantly lower than that in the comparative examples, indicating that the compositions of Examples 1-3 had inhibitory effect on the expression of ET-1 in HaCaT cells, and the inhibitory effect was significantly stronger than that in Comparative Examples 1-9. The expression of ET-1 in HaCaT cells in the group of the composition of Example 1 was significantly lower than that in Examples 2 and 3, indicating that according to the combination ratio in Example 1, the inhibitory effect on the expression of ET-1 in HaCaT cells was more significant.

In addition, by comparing the content of ET-1 in HaCaT cells in the groups of Examples 1-3 with that in the groups of Comparative Examples 1-6, it could be found that the content of ET-1 in HaCaT cells in the groups of Examples 1-3 was significantly lower than that in Comparative Example 6, which had the best effect among the comparative examples, and the difference was significant. It could thus be indicated that the effect was based on the complementary enhancement effect of the three, and that the effect was significantly superior to the effect of the extract of *Paeonia latiflora* Pall., *Dendrobium nobile* Lindl., or *Tricholoma matsutake* (S. Ito & S. Imai) Singer alone and the additive effect of a combination of any two of them.

Moreover, by comparing the content of ET-1 in HaCaT cells in the groups of Examples 1-3 with that in the group of Comparative Example 7, it could be found that the contents of ET-1 in HaCaT cells in the groups of Examples 1-3 were all significantly lower than that in Comparative Example 7, indicating that the inhibitory effects of the compositions of Examples 1-3 on the expression of ET-1 in HaCaT cells were significantly stronger than that of Comparative Example 7, indicating that only when used in combination at a specific ratio, *Paeonia latiflora* Pall., *Dendrobium nobile* Lindl. and *Tricholoma matsutake* (S. Ito & S. Imai) Singer can play an effective complementary enhancement effect, rather than a single additive effect.

Furthermore, according to the comparison of the content of ET-1 in HaCaT cells in the groups of Examples 1-3 with that in the groups of Comparative Examples 8 and 9, it could be found that small changes in the preparation process would also greatly affect the use effect of the final composition, and the extracts obtained under the specific technological conditions in the above examples could exert an effective complementary enhancement effect only after being prepared into a composition. The main active ingredients in the composition included paeoniflorin, matsutakeol, matsutakeol polysaccharide, dendrobium polysaccharide, etc.

**Test Example 2: Use of product containing composition in improving skin brightness**

In order to test the skin brightness enhancement effect of the composition, the inventors prepared a base emulsion as a substrate and tested the effect thereof on the skin of mice by compounding the compositions of the above examples and comparative examples.

In this test example, the formula of the base emulsion used was as shown in Table 2:

Table 2 Formula table of base emulsion

| Name | Content | Function |
| --- | --- | --- |
| Squalane | 3.9 g | Oil phase |
| Montanov S | 2.1 g | Surfactant |
| Glycerol | 2.4 g | Aqueous phase, humectant |
| Ethylparaben | 0.03 g | Preservative |
| Compositions in examples or comparative examples | 0.3 g | Main drug |
| Distilled water | Making up to 30 g | |

The preparation method for the base emulsion in Table 2 was as follows: squalane and MontanovS were weighed according to Table 2, mixed, stirred, heated and melted as the oil phase. Subsequently, the composition of the example or comparative example was dissolved in glycerol and a small amount of water (6.27 g in this example), as the first aqueous phase; and the remaining water (15 g in this example) was then heated to 90°C and ethyl paraben was then added as the second aqueous phase. The first aqueous phase was slowly dropwise added to the oil phase, and the second aqueous phase was then slowly dropwise added while stirring to obtain the base emulsion (the finished product was cream-like).

The example was Example 1, and the comparative example was Comparative Examples 1-3.

The specific test steps were as follows:

The experimental animals were female C57 mice aged 6 weeks, weighing 20-25 g. After one week of adaptive feeding, they were tagged with ear tags two days before the formal experiment. The back of the experimental mice was shaved 2 cm × 4 cm, depilated with a depilatory cream and rinsed clean with warm water, and the skin on the back of the mice was ensured to be free from damage.

Except the blank group, the model group and each experimental group were both modeled by means of an ultraviolet cross-linker. The UVB irradiation tube was 15 cm away from the back, the ultraviolet wavelength was 280-320 nm, and the daily irradiation doses in the first, second, third and fourth weeks were 300, 300, 400 and 500 mJ/cm$^2$, respectively, once every other day. From the second week, the experimental group was topically administered with the base emulsions containing the compositions of the examples and comparative examples once a day (specifically, administered 3 h after irradiation), and the whole experimental period was 28 days (four weeks). Each group was shaved once every 3 days before administration so as to avoid new hair interfering with the experimental results. The base emulsion in the model group did not contain any composition of the examples or comparative examples, and instead, the same amount of water was used. The blank group was not treated. Each group had six experimental mice.

Indicators were detected according to the following methods:

1) Skin brightness value:

Before modeling and 4 weeks after the administration was started, the skin color change was separately detected by means of a color difference meter, and the skin brightness value (L) was recorded.

The results were as shown in Table 3.

Table 3 L value and ΔL of brightness color difference on the back of mice

| Grouping | Brightness L on d0 | Brightness L on d28 | ΔL |
|---|---|---|---|
| **Blank group** | 58.42 | 58.97 | 0.55** |
| **Model group** | 58.56 | 56.47 | -2.09 |
| **EXAMPLE 1** | 58.76 | 58.98 | 0.22*** |
| **COMPARATIVE EXAMPLE 1** | 58.45 | 57.88 | -0.57*Δ |
| **COMPARATIVE EXAMPLE 2** | 58.79 | 58.15 | -0.63*Δ |
| **COMPARATIVE EXAMPLE 3** | 58.93 | 58.35 | -0.59**Δ |

ΔL was the difference value of the L value on d28 minus the L value on d0. Each group was subjected to one-way ANOVA relative to the model group, and P values (*$P < 0.05$, **$P < 0.01$, and ***$P < 0.001$) were calculated for significant difference analysis. Example 1 and Comparative Examples 1-3 were subjected to significance analysis, and P values were calculated (Δ$P < 0.05$, ΔΔ$P < 0.01$, and ΔΔΔ$P < 0.001$).

From the results of skin brightness of the experimental mice in different treatment groups shown in Table 3, it could be seen that the skin brightness of the mice decreased significantly after UVB modeling, while the compositions of the examples and comparative examples could all function to improve the skin brightness. The effect of the composition of Example 1 on improving skin brightness (ΔL) was greater than that in the comparative example, and there was a significant difference between the example and the comparative example, indicating that the composition of Example 1 had a better effect on regulating skin brightness than the extract of *Paeonia latiflora* Pall., *Dendrobium nobile* Lindl., or *Tricholoma matsutake* (S. Ito & S. Imai) Singer alone, and when used in a base carrier, it could function to significantly improve the skin brightness.

2) Masson-Fontana staining:

After the experiment was completed, the mice were sacrificed, and a part of skin was taken from the back and fixed with 4% paraformaldehyde, followed by tissue embedding, slicing and staining. The staining method was melanin Masson-Fontana staining. Masson Fontana staining utilizes melanin's ability to reduce silver ammonia solution to metallic silver (i.e. argyrophilic reaction) to display melanin. After staining, melanin would appear black. Image Pro Plus 6 software was used to calculate the integrated optical density (IOD) of melanin in the epidermis in the Masson-Fontana staining picture, so as to analyze the melanin content in the epidermis in different groups.

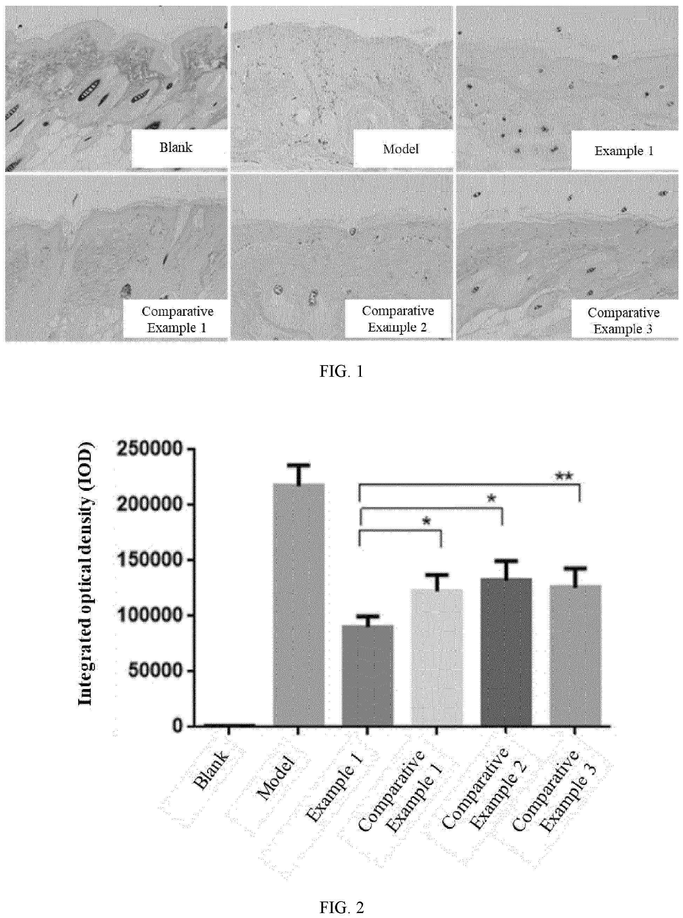

The results were as shown in Figs. 1 and 2.

Fig. 1 is the staining diagram (200×) of melanin in the skin of the back of mice in different groups after 4 weeks. It could be found that after UVB modeling, the thickness of the skin epidermis of the mice increased and the distribution of melanin in the epidermal layer increased. The use of emulsions containing the compositions of the examples and comparative examples could inhibit epidermal thickening and reduce the melanin content. However, by comparing Example 1 with Comparative Examples 1-3, it could be found that the content of epidermal melanin in Example 1 was significantly less than that in Comparative Examples 1-3, and there was also a significant difference in the integrated optical density (IOD) value of melanin between Example 1 and each comparative example (Fig. 2), indicating that the compositions of these examples had better effects on regulating skin melanin than the extract of *Paeonia latiflora* Pall., *Dendrobium nobile* Lindl., or *Tricholoma matsutake* (S. Ito & S. Imai) Singer alone, and when used in a base carrier, it could function to significantly suppress melanin synthesis.

3) Detection of the contents of ET-1 and TYR (tyrosinase) by ELISA:

A part of skin tissue (about 100 mg) was taken from the back of the experimental mice, washed with PBS to remove blood stains, cut into small pieces and put into a tissue grinder, and 1 mL of tissue lysate (Thermo Fisher Scientific Inc.) was added to make a homogenate. The tissue homogenate was centrifuged for 5 min at 2-8°C and 15000 r/min. The supernatant was taken, the protein concentration in the sample of each group was determined by means of a BCA kit, and the contents of tyrosinase (TYR) related to melanin synthesis in the supernatant and endothelin ET-1 were detected by means of an ELISA kit.

The results were as shown in Table 4.

Table 4 Expression of ET-1 and TYR in skin tissue of each group

| Grouping | ET-1 content (ng/mg) | TYR content (ng/mg) |
|---|---|---|
| **Blank group** | 1.98±0.38**** | 114.17±8.37*** |
| **Model group** | 4.10±0.30 | 152.48±11.94 |
| **EXAMPLE 1** | 2.35±0.15*** | 109.38±3.53*** |
| **COMPARATIVE EXAMPLE 1** | 3.09±0.15**$^\Delta$ | 130.35±1.11*$^{\Delta\,\Delta}$ |
| **COMPARATIVE EXAMPLE 2** | 3.52±0.34$^{\Delta\,\Delta}$ | 138.32±4.46$^{\Delta\,\Delta\,\Delta}$ |
| **COMPARATIVE EXAMPLE 3** | 3.27+0.12*$^{\Delta\,\Delta}$ | 139.46±3.62$^{\Delta\,\Delta\,\Delta}$ |

Each group was subjected to one-way ANOVA relative to the model group, and P values (*P < 0.05, **P < 0.01, and ***P < 0.001) were calculated for significant difference analysis. Example 1 and Comparative Examples 1-3 were subjected to significance analysis, and P values were calculated ($^\Delta$P < 0.05, $^{\Delta\,\Delta}$P < 0.01, and $^{\Delta\,\Delta\,\Delta}$P < 0.001).

From the expression levels of ET-1 and TYR in different groups shown in Table 4, it could be found that the contents of ET-1 and TYR in the skin tissues of the examples group were both lower than those of each comparative example, and the difference was significant, indicating that the compositions of the examples had better effects on the inhibitory regulation of ET-1 than the extract of *Paeonia latiflora* Pall., *Dendrobium nobile* Lindl., or *Tricholoma matsutake* (S. Ito & S. Imai) Singer alone. When used in a base carrier, it could function to significantly suppress the expression of ET-1 and TYR.

## EXAMPLE 4

In this example, the preparation methods for the extract of *Paeonia latiflora* Pall. and the extract of *Tricholoma matsutake* (S. Ito & S. Imai) Singer were the same as in Example 1. The preparation method for the extract of *Dendrobium nobile* Lindl. was as follows: dried *Dendrobium officinale* was taken, crushed, and sieved through an 80-mesh sieve, and yeast extract powder, magnesium sulfate heptahydrate, manganese sulfate, Tween-80 and purified water were added, such that the final concentration of *Dendrobium nobile* Lindl. in the mixed solution was 40 g/L, the final concentration of the yeast extract was 10 g/L, the final concentration of magnesium sulfate heptahydrate was 0.58 g/L, the final concentration of manganese sulfate was 0.25 g/L, and the final concentration of Tween-80 was 0.1% (v/v). The solution was adjusted to a pH value of 7.0 and heated at 115°C for 20 min for sterilization to obtain a *Dendrobium nobile* Lindl. raw material fermentation medium. *Lactobacillus reuteri* CCFM8631 at a final concentration of $1.0\times10^6$ cfu/mL was inoculated into the *Dendrobium nobile* Lindl. raw material fermentation medium and fermented at a constant temperature of 37°C and a constant pH value of 7.0 for 16 h, so that the viable bacteria count reached $5.0\times10^8$ cfu/mL. Fermentation was then continued for 8 h to obtain the extract of *Dendrobium nobile* Lindl. (*Dendrobium nobile* Lindl. fermentation broth).

The extract of *Paeonia latiflora* Pall., the extract of *Dendrobium nobile* Lindl. and the extract of *Tricholoma matsutake* (S. Ito & S. Imai) Singer were then uniformly mixed at a mass ratio of 2 : 2 : 1 to obtain a composition.

## EXAMPLE 5

This example was prepared according to the method in Example 4. In this example, the final concentration of *Dendrobium nobile* Lindl. in the mixed solution was 80 g/L, the final concentration of the yeast extract powder was 25 g/L, the final concentration of magnesium sulfate heptahydrate was 0.58g/L, the final concentration of manganese sulfate was 0.25g/L, and the final concentration of Tween-80 was 0.1% (v/v).

## TEST EXAMPLE 3

Taking the composition of Example 4, the extract of *Paeonia latiflora* Pall. prepared in Example 4, the composition of extract of *Dendrobium nobile* Lindl. and extract of *Tricholoma matsutake* (S. Ito & S. Imai) Singer (the extract of *Dendrobium nobile* Lindl. was the extract of *Dendrobium nobile* Lindl. prepared in Example 4, and the mixing mass ratio of the two extracts was 1 : 1), and the composition of extract of *Dendrobium nobile* Lindl. and extract of *Paeonia latiflora* Pall. (the extract of *Dendrobium nobile* Lindl. was the extract of *Dendrobium nobile* Lindl. prepared in Example 4, and the mixing mass ratio of the two extracts was 1 : 1) as test samples, the effects thereof on the content of ET-1 in HaCaT cells were detected. The test method was the same as in Test Example 1.

The results were as shown in Table 5.

Table 5: Inhibitory effect of composition on expression of ET-1 in skin keratinocytes HaCaT

| Grouping | Composition (mass ratio) | ET-1 content (pg/mg) | Inhibition rate (%) |
|---|---|---|---|
| Control group | \ | 9.89±0.257 | \ |
| Extract of *Dendrobium nobile* Lindl. prepared in Example 4 | Containing *Dendrobium nobile* Lindl. alone | 7.80±0.87 | 21.03 |
| Composition of extract of *Dendrobium nobile* Lindl. and extract of *Tricholoma matsutake* (S. Ito & S. Imai) Singer | *Dendrobium nobile* Lindl. : *Tricholoma matsutake* (S. Ito & S. Imai) Singer = 1 : 1 | 7.56±0.44 | 23.52 |
| Composition of extract of *Dendrobium nobile* Lindl. and extract of *Paeonia latiflora* Pall. | *Paeonia latiflora* Pall. : *Dendrobium nobile* Lindl. = 1 : 1 | 6.93±0.38* | 29.83 |
| EXAMPLE 4 | *Paeonia latiflora* Pall. : *Dendrobium nobile* Lindl. : *Tricholoma matsutake* (S. Ito & S. Imai) Singer = 2 : 2 : 1 | 5.19±1.90*** | 47.51 |

Compared with the control group, *P < 0.05, **P < 0.01, and ***P < 0.001.

From the above results, it could be seen that the extract of *Dendrobium nobile* Lindl. prepared by fermentation also had a certain inhibitory effect on ET-1 expression, and the inhibitory effect of the composition obtained by combining the extract of *Tricholoma matsutake* (S. Ito & S. Imai) Singer with the extract of *Paeonia latiflora* Pall. on the expression of ET-1 could reach 47.51%, which was a very significant effect. The effect of Example 5 was similar to that of Example 4.

The above examples are preferred embodiments of the present disclosure. However, the embodiments of the present disclosure are not limited by the above examples, and any other variations, modifications, substitutions, combinations and simplifications made without departing from the spiritual essence and principle of the present disclosure shall all be equivalent replacements and are all included in the scope of protection of the present disclosure.

## Claims

1. A composition with endothelin antagonism, comprising an extract of *Paeonia latiflora* Pall. or *Paeonia veitchii* Lynch, an extract of *Dendrobium,* and an extract of *Tricholoma matsutake* (S. Ito & S. Imai) Singer.

2. The composition according to claim 1, wherein a mass ratio of the extract of *Paeonia latiflora* Pall. , the extract of *Dendrobium,* and the extract of *Tricholoma matsutake* (S. Ito & S. Imai) Singer is (1-5) : (1-5) : (1-3).

3. The composition according to claim 2, wherein a mass ratio of the extract of *Paeonia latiflora Pall.* , the extract of *Dendrobium,* and the extract of *Tricholoma matsutake* (S. Ito & S. Imai) Singer is (1.5-3) : (1.5-3) : (1-2).

4. The composition according to any one of claims 1-3, wherein the *Dendrobium* comprises *Dendrobium nobile* Lindl. and *Dendrobium officinale.*

5. The composition according to any one of claims 1-3, wherein a preparation method for the extract of *Paeonia latiflora* Pall. comprises soaking a raw material of *Paeonia latiflora* Pall. in a 70-95% ethanol aqueous solution, and performing reflux extraction at 60-85°C to obtain the extract of *Paeonia latiflora* Pall.,
wherein a material-to-liquid ratio of the raw material of *Paeonia latiflora* Pall. to the 70-95% ethanol aqueous solution is 1 mg : 10-20 mL.

6. The composition according to any one of claims 1-3, wherein a preparation method for the extract of *Dendrobium.* comprises soaking a raw material of *Dendrobium* in water, performing reflux extraction at 95-100°C to obtain a extraction product, filtering the extraction product to obtain a filtrate, and freeze-drying the filtrate to obtain the extract of *Dendrobium,*

wherein a material-to-liquid ratio of the raw material of *Dendrobium nobile* to water is 1 mg : 10-20 mL; or

mixing the raw material of *Dendrobium* with *Lactobacillus sp.* for fermentation to obtain the extract of *Dendrobium*.

7.  The composition according to any one of claims 1-3, wherein a preparation method for the extract of *Tricholoma matsutake* (S. Ito & S. Imai) Singer comprises:

    subjecting a raw material of *Tricholoma matsutake* (S. Ito & S. Imai) Singer to reflux extraction in a 40-70% ethanol aqueous solution at 40-80°C to obtain the extract of *Tricholoma matsutake* (S. Ito & S. Imai) Singer, wherein a material-to-liquid ratio of the raw material of *Tricholoma matsutake* (S. Ito & S. Imai) Singer to the 40-70% ethanol aqueous solution is 1 mg : 10-30 mL.

8.  The composition according to any one of claims 1-7 for use in inhibiting endothelin.

9.  The composition according to any one of claims 1-7 for use in brightening and whitening skin.

10. A food, medicament or cosmetic product, comprising the composition according to any one of claims 1-7.

11. The food, medicament or cosmetic product according to claim 10, further comprising an auxiliary material.

12. The composition according to any one of claims 1-7 for use as a medicament.

13. The composition according to any one of claims 1-7 for use in reducing melanin or tyrosinase in skin.

14. The composition according to any one of claims 1-7 for use in preventing and/or ameliorating a disease in which endothelin is taken as a target.

15. The composition for use according to claim 14, wherein the disease is skin endothelin regulation-associated problems comprising speckle, scleroderma, psoriasis, and wound healing, and diseases comprising cardiovascular and cerebrovascular diseases, tumors, and pulmonary arterial hypertension.

FIG. 1

FIG. 2

Europäisches
Patentamt

European
Patent Office

Office européen
des brevets

## EUROPEAN SEARCH REPORT

Application Number

EP 23 19 2545

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 113 499 290 A (GUANGZHOU UNIASIA COSMETICS TECH CO LTD) 15 October 2021 (2021-10-15) * the whole document * | 1-15 | INV. A61K8/00 A61K36/07 A61K36/65 A61K36/8984 |
| X | BEIJING DR PLANT BIOTECHNOLOGY CO ET AL: "DR PLANT PLATEAU TRICHOLOMA MATSUTAKE FRESH BRIGHTEN CREAM", INTERNET CITATION, 16 March 2021 (2021-03-16), XP009542613, Retrieved from the Internet: URL:https://hzpba.nmpa.gov.cn/gccx/chakanH is.html?prodId=20210312171702vdd29&gb=G * the whole document * | 1-15 | A61P17/00 A61P17/02 A61P17/06 |
| X | CN 107 897 908 A (LUOYANG SHENSHAN BIOTECHNOLOGY CO LTD) 13 April 2018 (2018-04-13) * the whole document * | 1-15 | |
| X | CN 107 080 716 A (GUANGXI RONGXIAN TIANSHUN DENDROBIUM CO LTD) 22 August 2017 (2017-08-22) * the whole document * | 1-15 | **TECHNICAL FIELDS SEARCHED (IPC)** A61K A61Q A61P |
| X | DATABASE GNPD [Online] MINTEL; 18 January 2021 (2021-01-18), anonymous: "Tian Xiang Que Wu Shou Yu Essence", XP093119624, Database accession no. 8369623 * abstract * | 1-15 | |

The present search report has been drawn up for all claims

| Place of search | Date of completion of the search | Examiner |
|---|---|---|
| Munich | 17 January 2024 | Friederich, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 19 2545

This annex lists the patent family members relating to the patent documents cited in the above-mentioned European search report.
The members are as contained in the European Patent Office EDP file on
The European Patent Office is in no way liable for these particulars which are merely given for the purpose of information.

17-01-2024

| Patent document cited in search report | | Publication date | Patent family member(s) | | Publication date |
|---|---|---|---|---|---|
| CN 113499290 | A | 15-10-2021 | CN | 113499290 A | 15-10-2021 |
| | | | WO | 2023284221 A1 | 19-01-2023 |
| CN 107897908 | A | 13-04-2018 | NONE | | |
| CN 107080716 | A | 22-08-2017 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2139317